# EUROPÄISCHE PATENTSCHRIFT

(11) **EP 0 833 153 B1**
(45) Veröffentlichungstag und Bekanntmachung des Hinweises auf die Patenterteilung: **07.06.2006**
(21) Anmeldenummer: 97115223.6
(22) Anmeldetag: 03.09.1997
(51) Int. Cl.: G01N 33/557, G01N 21/82

(54) **Verfahren zur instrumentellen Bestimmung einer sich zeitabhängig ändernden Messgrösse**
Method for the instrumental determination of a time-dependent variable
Méthode pour la détermination instrumentale d'une variable dépendante du temps

(30) Priorität: 28.09.1996 DE 19640121
(43) Veröffentlichungstag der Anmeldung: 01.04.1998
(73) Patentinhaber: Dade Behring Marburg GmbH, 35001 Marburg (DE)
(72) Erfinder: Patzke, Jürgen, Dr., 35039 Marburg (FR)

(56) Entgegenhaltungen:
- EP-A- 0 254 430
- WO-A-87/03960
- DE-A- 2 529 117
- GB-A- 1 600 139
- US-A- 4 063 817
- US-A- 4 224 304
- US-A- 4 472 505

## Beschreibung

Die vorliegende Erfindung betrifft ein Verfahren zur instrumentellen Bestimmung des maximalen Reaktionsgeschwindigkeit aus einer sich zeitabhängig mit der Reaktion ändernden Meßgröße L(t) in einem Bereich mit linearem Reaktionsverlauf, wobei der Zeitpunkt sowie die Größe des Reaktionszeitfensters mit dem linearen Bereich variabel sind und von der Art der Reaktion und den Reaktionsbedingungen abhängen. Speziell betrifft die vorliegende Erfindung die Bestimmung von Protein-Konzentrationen mit Hilfe einer von spezifischen Antikörpern erzeugten Lichtstreuung in homogenen Lösungen. Insbesondere betrifft die Erfindung langsam ablaufende Reaktionen, die über längere Zeit weitgehend linear verlaufen, wobei als Meßgröße die Geschwindigkeit der Bildung von Antigen-Antikörper-Komplexen in dem linear verlaufenden Abschnitt der Reaktion ermittelt wird.

Das Phänomen der Lichtstreuung an Teilchen, die sich in einem homogenen Medium befinden, wird sowohl über die Messung der Streulichtintensität (Nephelometrie) als auch über die Messung des Intensitätsverlustes des durch das Medium tretenden Lichtstrahls (Turbidimetrie) zur Konzentrationsbestimmung genutzt.

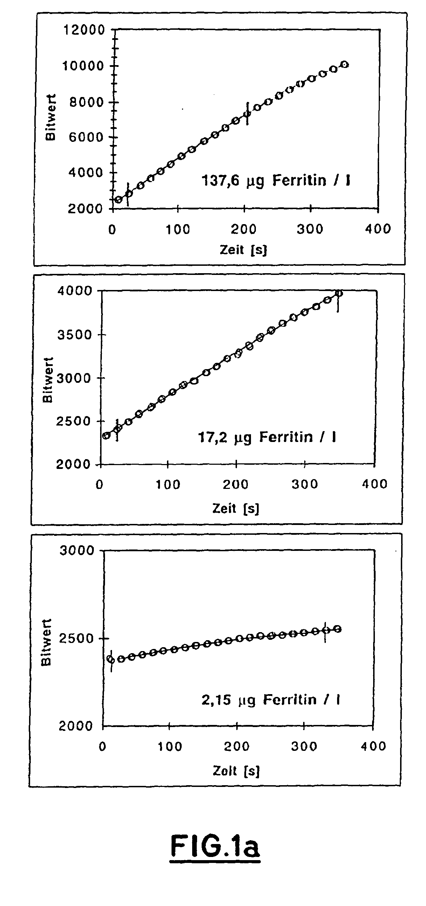

Die immunchemische Reaktion zwischen einem löslichen Antigen und einem bi- oder polyvalenten Antikörper führt zu großen und stark lichtstreuenden Molekülaggregaten. Der zeitliche Verlauf solcher Reaktionen entspricht sehr häufig dem allgemeinen kinetischen Verlauf aufeinanderfolgender Reaktionen 1. Ordnung und zeigt einen Wendepunkt und somit tritt die maximale Reaktionsgeschwindigkeit erst im Laufe der Reaktion auf (siehe z. B. Fig. 1a-1c). Aus den Signal-Zeit-Kurven in Fig. 1a-1c kann auf verschiedene Weise ein konzentrationsabhängiges Meßsignal gewonnen werden.

Die Intensität der Signaländerung kann dadurch verstärkt werden, daß einer der Reaktionspartner an Partikel gebunden ist, z. B. in den dem Fachmann an sich bekannten "partikelverstärkten Assays".

Im Endpunktverfahren wird das Meßsignal zu einem so späten Zeitpunkt ermittelt, bei dem es sich erfahrungsgemäß nicht mehr verändert, aber keine Präzipitation stattfindet. Beim Verfahren "Fixed time" ist das eigentliche Meßverfahren die Differenz zweier Signale, die zu verschiedenen, aber fest vorgegebenen Zeitpunkten ermittelt werden.

Beim kinetischen Verfahren "Peak rate method" wird die maximale Reaktionsgeschwindigkeit (V_{Max}), d. h. die maximale Änderung (δ) des Signals (S) pro Zeiteinheit (δ t) ermittelt
a) durch Messungen von δ S in hinreichend kurzen Zeitintervallen (δ t) und Bestimmung des größten Quotienten δ S/δ t,
b) elektronische Differenzierung δ S/δ t und Bestimmung des Maximums,
c) Konstruktion der Tangente an die Signal/Zeit-Kurve und Bestimmung der maximalen Steigung S = Signal t = Zeit.

Die US 4 472 505 offenbart ein Verfahren gemäß dem Oberbegriff des Ansprüche 1 and 2.

Das erfindungsgemäße Verfahren kann prinzipiell auf alle Bestimmunge der maximalen Reaktionsgeschwindigkeit aus einer sich zeitabhängig mit der Reaktion ändernden Meßgröße angewendet werden, wenn die Änderung der Meßgröße nur in einem Teilbereich linear ist und zur Auswertung der lineare Teil herangezogen werden soll.

Eine große Zahl von Analyten kann heute nach den aufgeführten Verfahren mit direkter oder indirekter Streulichtmessung quantifiziert werden. Betrachtet man die Abhängigkeit eines geeigneten Meßsignals von der Konzentration eines Reaktionspartners, z. B. des Antigens, während der andere Reaktionspartner mit konstanter Konzentration eingesetzt wird, so kann man zum Beispiel bei immunchemischen Reaktionen beobachten, daß das gleiche Meßsignal sowohl auf eine niedrige als auch auf eine hohe Konzentration des Analyten zurückgeführt werden kann. Dies führt zu einer Zweideutigkeit der Signal-Konzentration-Beziehung die dem Fachmann als Antigenüberschuß-Phänomen "high-dose hook" oder Heidelbergerkurve bekannt ist. Diese Zweideutigkeit ist prinzipiell überall dort beobachtbar, wo Komplexe unterschiedlicher Stöchiometrie möglich sind, je nach Überschuß des einen oder anderen Reaktionspartners, und sich diese Komplexe in ihrer Signaleigenschaft, zum Beispiel Streulicht, nicht unterscheiden.

Solche immunchemische Bestimmungsverfahren sind dem Fachmann an sich bekannt, z. B. aus der EP 0 252 127.

Neben der möglichen Zweideutigkeit der Signal-Konzentration-Beziehung, ist ein weiteres Problem die Ermittlung niedriger Konzentrationen, bzw. die Auswertung langsam verlaufender Reaktionen. Der Reaktionsverlauf der Antigen-Antikörper-Bindung zeigt prinzipiell eine lag-Phase zu Beginn, einen Bereich maximaler Reaktionsgeschwindigkeit und einen Sättigungsbereich (Fig. 1a-1c). Die Ausprägung dieser drei Phasen ist sehr stark abhängig von der Konzentration des Antigens, des Antikörpers und weiterhin von vielen anderen Faktoren, wie z. B. der Temperatur und dem Verdünnungsmedium, die in einem Testsystem jedoch möglichst konstant gehalten werden.

Der vorliegenden Erfindung lag somit das technische Problem zugrunde, ein immunchemisches Bestimmungsverfahren mit Hilfe von durch spezifischen Antikörpern erzeugte Lichtstreuung zu schaffen, welches sowohl die Messung sehr niedriger Konzentrationen erlaubt als auch eine hohe Sicherheit gegen den "high-dose hook" Effekt bietet.

Die Lösung dieses technischen Problems erfolgt durch die Bereitstellung der in den Ansprüchen beschriebenen Ausführungsformen.

Wesentlicher Bestandteil des erfindungsgemäßen Verfahrens ist, daß durch geeignete technische Schritte das Meßzeitfenster der jeweiligen Reaktionskinetik so angepaßt wird, daß die Auswertung zuverlässig im linearen Bereich und im Bereich der maximalen Reaktionsgeschwindigkeit der zeitabhängigen Reaktion erfolgt. Das Ergebnis einer solchen Auswertung ist die maximale Reaktionsgeschwindigkeit V_{MaxLin}, gelegentlich auch als X_{Lin} bezeichnet.

Das erfindungsgemäße Verfahren kann durch unterschiedliche technische Ausführungsformen gewährleistet werden.

Analyte im Sinne der Erfindung sind Plasmaproteine, wie z. B. Ferritin, PSA, IgA, IgG und auch Proteine, die dem Bereich der Gerinnung zuzuzählen sind, wie z. B. D-Dimer und Gerinnungsfaktoren, insbesondere genetische Varianten von Gerinnungfaktoren und ferner Haptene, wie z. B. Hormone und Messengerpeptide.

Vorteilhaft ist auch die Anwendung des erfindungsgemäßen Verfahrens auf die Bestimmung der Funktionsfähigkeit des Gerinnungssystems, wie z. B. Quick-Test und aPTT.

So wurde überraschenderweise festgestellt, daß das im folgenden beschriebene Bestimmungsverfahren sowohl die Messung niedriger Konzentrationen erlaubt als auch eine erhöhte Sicherheit gegen den "high-dose hook" Effekt gewährleistet.

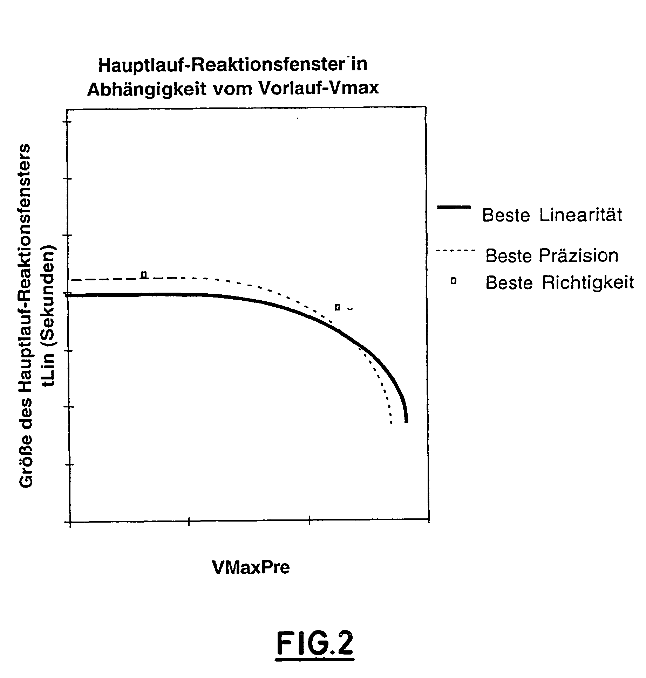

Die Durchführung des erfindungsgemäßen Verfahrens erfolgt in zwei Stufen, wobei eine Probe gemessen und die gespeicherte Reaktionskinetik verwendet wird. In der ersten Stufe wird mittels eines relativ kurzen Zeitfensters die vorläufige maximale Reaktionsgeschwindigkeit ermittelt. Die Länge des Zeitfensters t_{Test} wird vorzugsweise so eingestellt, daß im Mittel über mehrere Messungen und Chargen es der Länge des linearen Reaktionsabschnittes bei der Reaktionskinetik von Proben, deren Konzentration am oberen Ende des Meßbereichs liegen, entspricht. Es wird daraus auch deutlich, daß die Größe des Vorlaufreaktionsfensters ein testspezifischer Parameter ist, der unter anderem von dem Analyten aber auch dem verwendeten Testsystem, bzw. Nachweissystem abhängig ist. Aus der so ermittelten V_{MaxPre} wird das optimale Reaktionszeitfenster ermittelt. Die Abhängigkeit zwischen V_{MaxPre} und dem optimalen Reaktionszeiter muß in Vorversuchen testspezifisch bestimmt werden (Fig. 2). Wichtig ist dabei, daß für jede V_{MaxPre} ein Reaktionsfenster ermittelt wird, das einerseits möglichst viele Meßpunkte einbezieht, andererseits aber auch nur Punkte berücksichtigt, die auf dem linearen Abschnitt der Reaktion liegen.

Zur Ermittlung dieser testspezifischen Abhängigkeit können verschiedene Verfahren eingesetzt werden. Wichtig ist dabei, daß die Abhängigkeit zwischen V_{maxPre} und Reaktionszeitfenstergröße so festgelegt wird, daß Präzision und Richtigkeit optimal sind. Für die Festlegung dieser Abhängigkeit können z. B. Messungen an einem Pool von Serumproben unterschiedlicher Analytkonzentration oder Serum-Standards in unterschiedlicher Verdünnung durchgeführt werden. Die Konzentrationen erstrecken sich dabei vorteilhafterweise über den gesamten gewünschten Meßbereich. In einem ersten Schritt werden alle Proben gemessen, die Kinetiken gespeichert und die Werte von V_{MaxPre} ermittelt. Für jede Probenmessung erhält man nun durch schrittweise Veränderungen des Reaktionsfensters unterschiedliche Korrelationskoeffizienten. Die Fenstergröße mit dem besten Korrelationskoeffizienten berücksichtigt auch am besten den linearen Abschnitt der Reaktion. Ebenfalls als Maß für die Linearität geeignet ist die durchschnittliche Entfernung der Meßpunkte von der Regressionsgeraden. So erhält man zuerst für jede Probe mit einem bestimmten V_{MaxPre} das für die Linearität beste Reaktionszeitfenster. Als Ergebnis für alle Proben mit unterschiedlichen V_{MaxPre} ergibt sich eine Beziehung zwischen V_{MaxPre} und der Hauptlaufreaktionsfenstergröße t_{Lin} für die beste Linearität im Reaktionsfenster. Figur 2 zeigt beispielhaft die Beziehung zwischen Hauptlaufreaktionsfenstergröße t_{Lin} und U_{MaxPre} bei bestem Korrelationskoeffizienten.

Diese Beziehung wird benutzt, um eine Referenzkurve mit Standardserum-Verdünnungsstufen bekannter Konzentration zu erstellen (V_{MaxPre} → Hauptlauf-Reaktionsfenstergröße → Hauptlauf-V_{Max})

Da nicht sichergestellt ist, daß das Kriterium Linearität auch die besten Ergebnisse bei Präzisionsmessungen und bei Kontrollwiederfindungen liefert, erfolgt dann eine Feineinstellung der Beziehung zwischen Hauptlauf-Reaktionsfenster und V_{MaxPre}. Hierbei wird die oben erstellt Referenzkurve nicht verändert, um nicht zu viele Parameter gleichzeitig zu variieren.

Für verschiedene Konzentrationsstufen von Proben, Standards und Kontrollen werden Inter- und Intra-Assay-Präzisionsmessungen (Intra-Assay = in der Serie, Inter-Assay = An verschiedenen Tagen) durchgeführt, wobei die Hauptlauf V_{Max}-Bestimmungen mit Reaktionsfenstern unterschiedlicher Größe durchgeführt werden. Als Ergebnis erhält man für jede Messung und damit für jedes V_{MaxPre} eine Hauptlauf-Reaktionsfenstergröße, die die beste Präzision liefert. Die Zusammenfassung aller Messungen ergibt eine ähnliche Abhängigkeit, wie bei der Linearität. Ein beispielhafter Verlauf ist in Fig. 2 angegeben.

Die prinzipiell gleiche Vorgehensweise bei der Messung von Kontrollmaterial ergibt die beste Richtigkeit (Fig. 2) Nun kann man sich für eine Zuordnung von Hauptiauf-Reaktionsfenstergröße t_{Lin} zu V_{MaxPre} -Werten entscheiden, die sowohl gute Präzisionen, als auch eine gute Wiederfindung von Kontroll-Sollwerten gewährleistet. Das Ergebnis für Ferritin ist in Tabelle 1 dargestellt.

Nach Festlegung dieser Relation zwischen t_{Lin} und V_{MaxPre} muß die Berechnung der Referenzkurve mit den neuen Kriterien wiederholt werden. Nun kann eine vollständige Charakterisierung der neuen Methode erfolgen, wie sie in Tabelle 1 am Beispiel Ferritin zusammengefaßt ist.

**Tabelle 1 Das V_{MaxLin}-Verfahren am Beispiel Ferritin**

| | | |
|---|---|---|
| V_{MaxLin}-Parameter für Ferritin: | | |
| Minimum-Reaktionsfenster: | 40 Sekunden | |
| Vorlauf-Reaktionsfenser | 80 Sekunden | |
| V_{MaxPre} | | t_{Lin} |
| > 25 Bit/s → | | 80 Sekunden |
| 20 - 25 Bit/s → | | 240-160 Sekunden |
| 10 - 20 Bit/s → | | 360-240 Sekunden |
| < 10 Bit/s → | | 360 Sekunden |

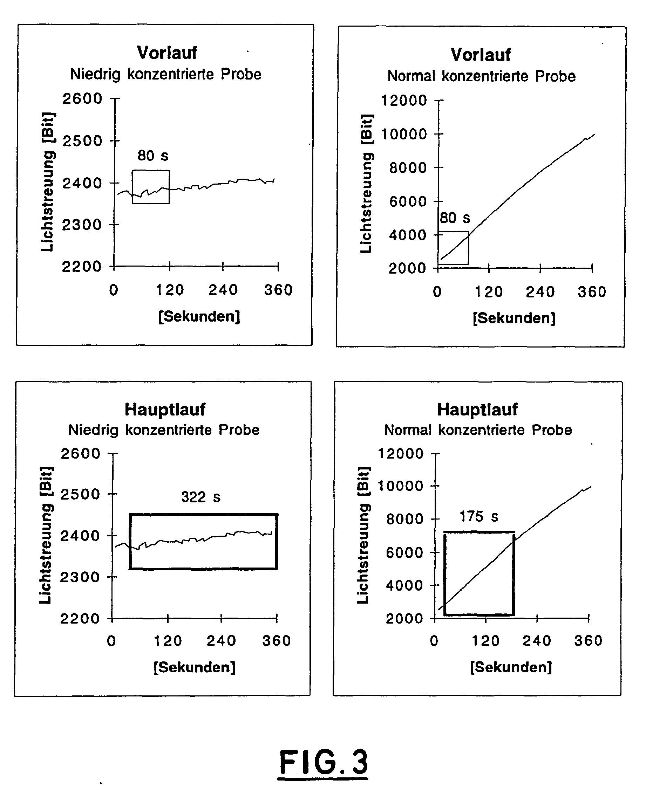

a) Niedrig konzentrierte Probe mit Minimun (Fig. 3) Minimum gefunden bei: 38 Sekunden
   V_{MaxPre}: 0,270 Bit/s (38 - 118 Sekunden)
   t_{Lin}: 360 Sekunden
   V_{MaxLin}: 0,123 Bit/s (38-360 Sekunden)
b) Normal konzentrierte Probe ohne Minimum (Fig. 3) Minimum gefunden bei: 0 Sekunden
   V_{MaxPre}: 24,02 Bit/s (0 - 80 Sekunden)
   t_{Lin}: 175 Sekunden
   U_{MaxLin}· 23,64 Bit/s (23 - 198 Sekunden)

Es ist zu betonen, das die so ermittelten Parameter sehr spezifisch für das Testsystem sind. Zu einem Testsystem gehören der Analyzer, die Meßvorschriften und die Reagenzien zur Bestimmung eines bestimmten Analyten, wenn notwendig mit Chargen-spezifischen Unterschieden, und natürlich das Probenmaterial.

Es ist auch möglich, für jede Messung die Linearität der Meßkurve nach mathematischen Linearitätskriterien zu bewerten und nur den Teil der Kurve auszuwerten, der diesen vorgegebenen Kriterien entspricht. Es ergeben sich dann jedoch gerade bei niedrigen Analytkonzentrationen schlechte Präzisionen.

Eine weitere Möglichkeit zur Bestimmung von V_{Max} wäre es, an die Meßkurve einer beliebigen Probe ein Polynom anzupassen und nachfolgend die erste Ableitung des Polynoms zu bilden. Das Maximum der ersten Ableitung gibt die maximale Reaktionsgeschwindigkeit an, allerdings hat sich gezeigt, daß insbesondere bei Reaktionen mit hoher Streuung wenig Verlaß auf ein derart ermitteltes V_{Max} ist, wie z. B. aus der Tabelle 2 (Polynom) zu ersehen ist.

**Tabelle 2: Bestimmung von V_{Max} direkt aus dem Polynom 3. Grades**

| **Verdünnung 1:** | **Konzentration [µg/l]** | **Polynom** | | **Integral** | |
|---|---|---|---|---|---|
| | | **MW [Bit/s]** | **VK [%]** | **MW [Bit/s]** | **VK [%]** |
| 2,5 | 137,60 | 25,50 | 2,0 | 25,24 | 2,0 |
| 5 | 68,80 | 18,09 | 3,4 | 17,65 | 3,4 |
| 10 | 34,40. | 9,61 | 4,8 | 9,44 | 4,9 |
| 20 | 17,20 | 4,91 | 3,3 | 4,61 | 3,0 |
| 40 | 8,60 | 2,45 | 7,8 | 2,11 | 2,2 |
| 80 | 4,30 | 1,19 | 7,6 | 0,98 | 4,8 |
| 160 | 2,15 | 0,58 | 16,3 | 0,46 | 3,5 |
| 320 | 1,08 | 0,34 | 38,6 | 0,22 | 9,7 |
| 640 | 0,54 | 0,17 | 48,6 | 0,09 | 15,0 |

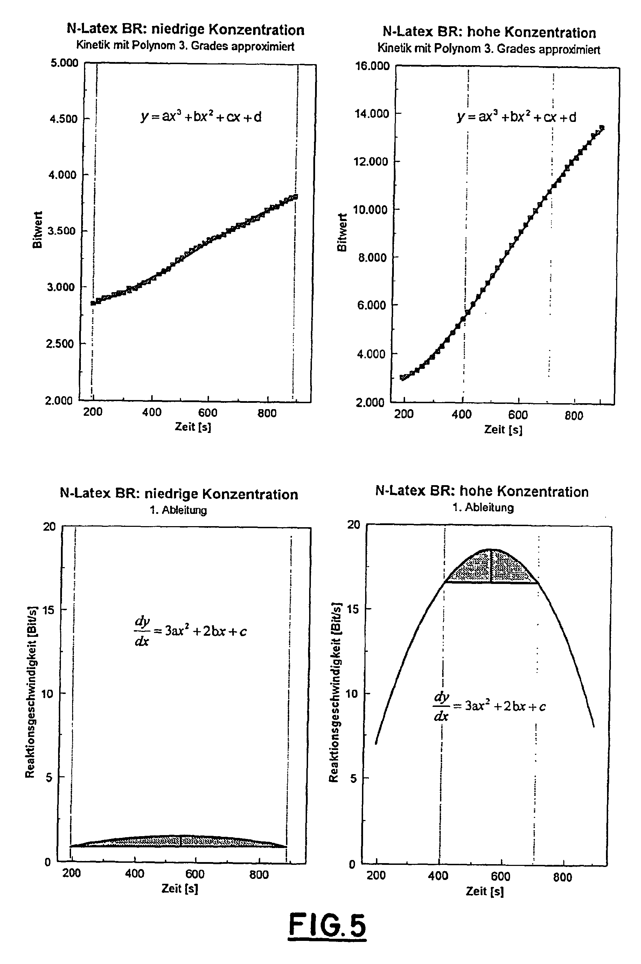

Überraschenderweise konnte gezeigt werden, daß durch Anpassen einer testspezifischen Integralfläche unterhalb der ersten Ableitung des Polynoms (siehe Figur 5) ein Meßzeitraum t_{Lin} bestimmt werden kann, in dem die Meßkurve über eine lineare Regression ausgewertet werden kann und zu einem Ergebnis führt, das in puncto Linearität, Präzision und Richtigkeit dem vorbeschriebenen Verfahren gleichwertig ist. Die Größe der Integralfläche ist testspezifisch, d. h. sie wird bevorzugterweise für jedes Testverfahren und jeden Analyten und gegebenenfalls auch für bestimmte Testgeräte empirisch ermittelt.

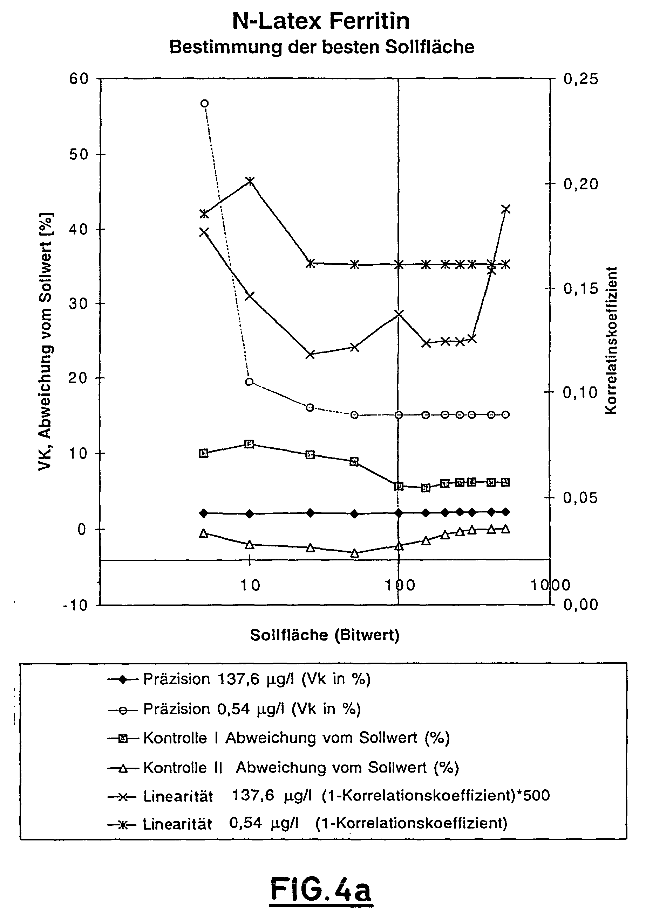

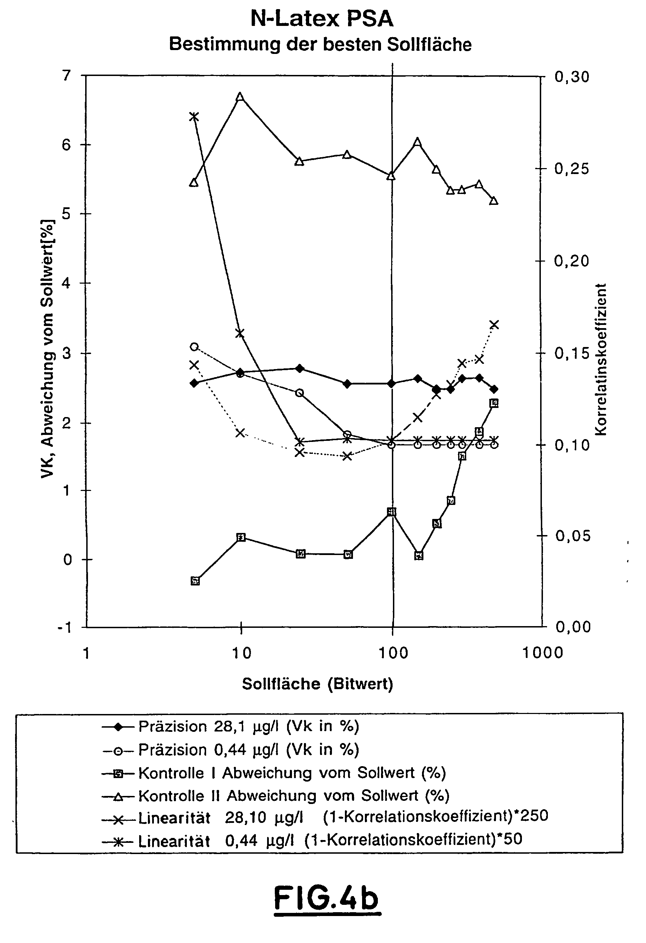

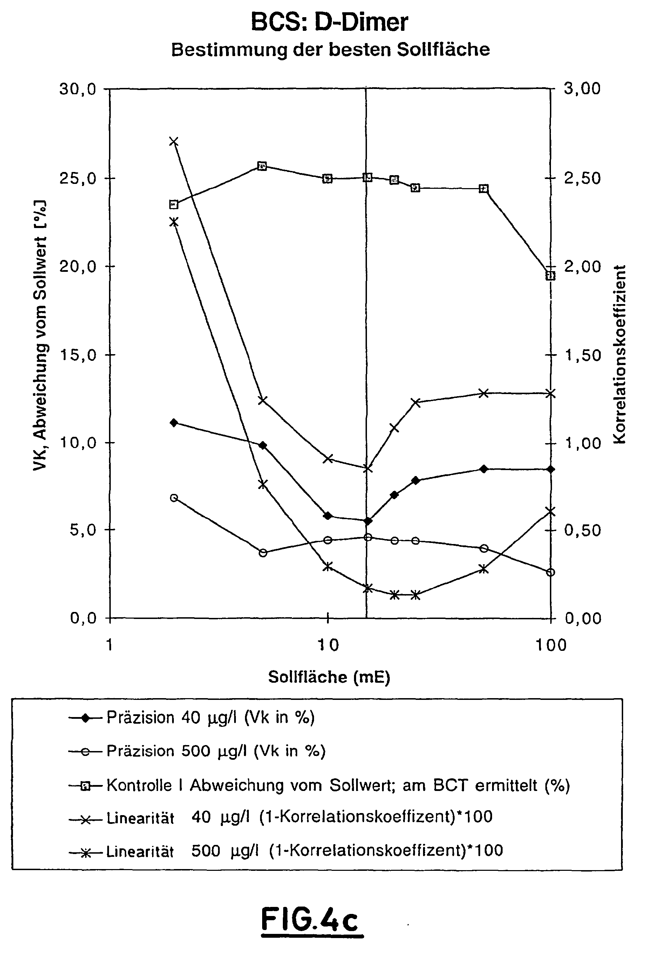

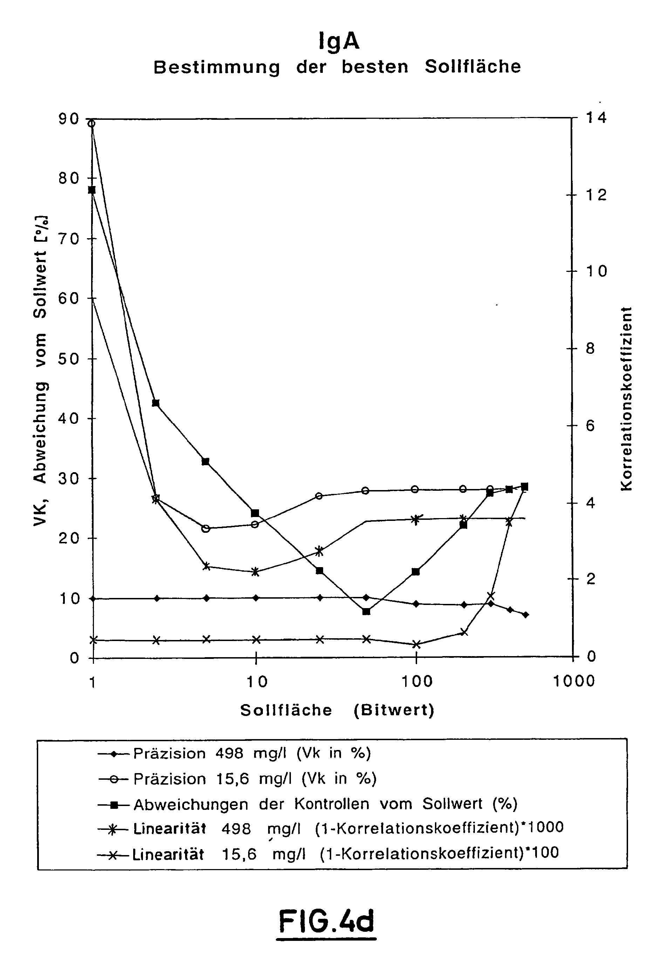

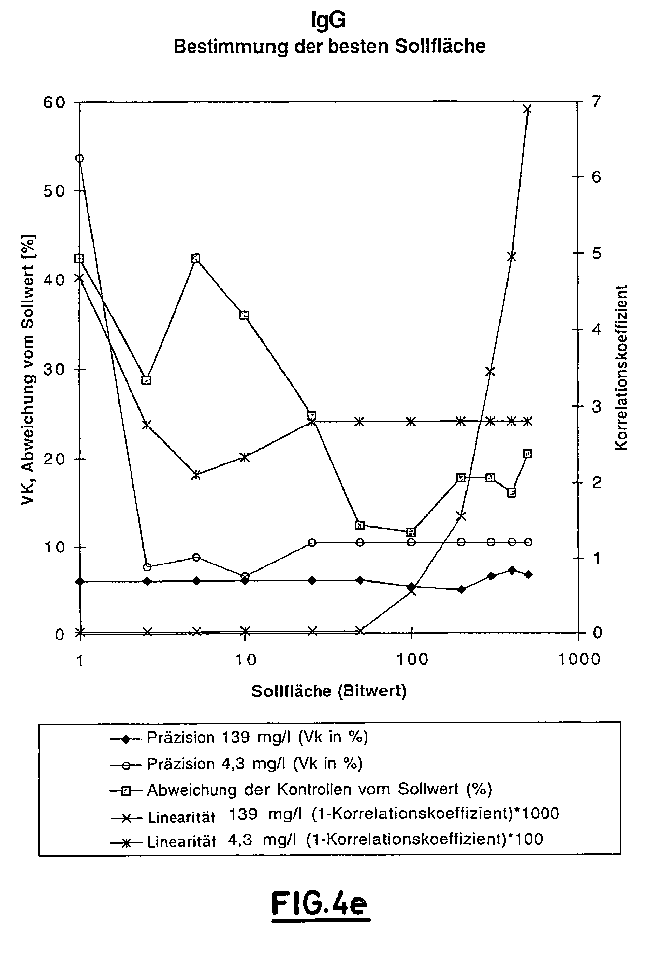

So zeigte sich, daß bei einer Integralfläche von 100 für verschiedene Latex-verstärkte Teste an einem Gerät (BN 11, Behringwerke AG, Marburg) optimale Ergebnisse erzielt werden konnten, z. B. für Ferritin und prostataspezifisches Antigen (Fig. 4 a und b). Bei dem gleichen Gerät ist die beste Integralfläche für nicht Latex-verstärkte Teste 10 (Fig. 4 d und e). Bei einem anderen Gerät (BCS, Behringwerke AG, Marburg) ist die beste Fläche für den Latex-verstärkten Test bei photometrischer Mesung 20 (Fig. 4 c).

Die empirische Bestimmung der optimalen Integralfläche erfolgt in einem getrennten Lauf wie die Festlegung von V_{maxPre} und t_{Lin} , jedoch ist für diese Variante die ermittelte Integralfläche eine Konstante, die für alle Konzentrationen gilt. In den Beispielen der Fig. 4 sind jeweils eine sehr hohe und sehr niedrige Konzentration angegeben. So wurden z. B. bei der Ermittlung der Präzision der verschiedenen Latex-verstärkten Testen am Behring-Nephelometer (Behringwerke AG, Marburg) für jeden Referenzpunkt Präzisionsmessungen in der Serie durchgeführt werden. Die Linearität ergab sich bei jeder Messung durch den Korrelationskoeffizienten der linearen Regression. Die Richtigkeit wurde durch die Messung von Kontrollseren und Faltung an einer Referenzkurve ebenfalls ausgewertet nach der Integralmethode ermittelt.

Die so ermittelte Integralfläche wird für jede Messung unterhalb der ersten Ableitung so eingepaßt, das nach oben die erste Ableitung des Polynoms begrenzend für die Fläche ist, nach unten gibt der Schnittpunkt der unteren Begrenzungsgeraden mit der Kurve auf der X-Achse an, wo der Beginn und das Ende des Meßzeitraums liegt.

Das Polynom wird nach dem Prinzip der kleinsten Quadrate aus den Wertepaaren der Reaktionskinetik berechnet. Das System von Normalgleichungen wird nach dem Gaus'schen Algorithmus gelöst, einem Verfahren, das dem Fachmann an sich zur Auslösung nicht linearer Regressionen bekannt ist. Vorteilhafterweise ist das angepaßte Polynom 3. Grades, aber auch Polynome höheren Grades können verwendet werden.

Die folgenden Beispiele erläutern die Erfindung:

### Vergleichsbeispiel :

### a) Peak Rate Methode

Das TurbiTimeSystem der Behringwerke ist ein gutes Beispiel für ein kinetisches Auswerteverfahren (DE 33 47 162). Es besitzt eine Meßkammer, in der jeweils in einer einzelnen Küvette die Reaktion abläuft. Die Messungen der optischen Dichte erfolgt solange, bis die maximale Reaktionsgeschwindigkeit V_{Max}, das Ergebnis der Messung, erreicht ist und wird dann abgebrochen.

Die Reagenzien für das System sind so eingestellt, daß sie eine schnelle Reaktion ermöglichen und im Idealfall bereits nach einigen Sekunden die Messung abgebrochen werden kann. Weiterhin wird der Zeitpunkt des Erreichens von V_{Max} ausgewertet. Dadurch kann eine Entscheidung getroffen werden, auf welcher Seite der Heidelberger Kurve man sich befindet und ein falsch zu niedriges Ergebnis kann nahezu ausgeschlossen werden.

### b) Fixed Time Methode

Die Nephelometer der Behringwerke (BN, BN100, BNII) messen die Zunahme der Trübung als Lichtstreuung. Die Auswertemethode wird auf Grund der festen Reaktionszeiten "Fixed time" genannt. Als Ergebnis der Messung wird die Trübungsdifferenz zwischen einem Anfangswert und einem Endwert ermittelt. Die Nephelometer sind vollautomatische Systeme, bei denen die Ansätze in vielen Küvetten gleichzeitig inkubieren, um einen hohen Durchsatz zu ermöglichen. Die Küvetten werden mit einem Rotor in regelmäßigen Abständen zur Meßwertaufnahme an der Meßoptik vorbeigeführt. Daher erhält man eine Reaktionskinetik mit Meßpunkten in relativ großen Abständen , z.B. 16 Sekunden beim BN II.

### Beispiel 1 -

### Das neue V_{MaxLin}-Verfahren mit 2 Stufen Auswertung

Das Verfahren ist zweistufig (Fig. 3, Tab. 1):

### 1. Ermittlung der optimalen Länge des Hauptlauf-Reaktionsfensters (t_{Lin})

In einem ersten Durchlauf wird mit einem kleinen Vorlauf-Reaktionsfenster über den Verlauf der gesamten Kinetik die maximale Reaktionsgeschwindigkeit V_{maxPre} gesucht. Die Größe des Fensters ist spezifisch für den jeweiligen Test und wird so festgelegt, daß es auch bei hohen Antigenkonzentrationen noch innerhalb des linearen Bereichs der Reaktion liegt. Die damit ermittelte Reaktionsgeschwindigkeit ist noch relativ ungenau.

Sie wird benutzt, um die ideale Größe des Hauptiauf-Reaktionsfensters (t_{Lin}) für diese Antigenkonzentration festzulegen. Ideal heißt hier, daß annähernd der gesamte Teil der Reaktion, der linear verläuft, zur Festlegung der Reaktionsgeschwindigkeit herangezogen wird. Die Verweistabelle über die Zuordnung von Reaktionsgeschwindigkeit und Größe des Hauptlauf-Reaktionsfensters wird ebenfalls testspezifisch empirisch ermittelt (Fig. 2, Tab. 1).

### 2. Ermittlung der maximalen Reaktionsgeschwindigkeit (V_{MaxLin})

### a) Minimumerkennung

Zu Beginn einer Kinetik kann es zu einem Absinken der Trübung kommen, bevor die eigentliche Reaktion startet. Dadurch würde bei einem großen t_{Lin} (z.B. 360 Sekunden) ein falsch zu niedriges V_{MaxLin} ermittelt werden. Dies verhindert die Minimumerkennung.

Zur Festlegung des Startzeitpunktes der Reaktion wird ein sehr kurzes Reaktionsfenster verwendet. Im Beispiel der Fig. 3 und Tab. 1 erfaßt es einen Zeitraum von 40 Sekunden. Das Reaktionsfenster startet zum Zeitpunkt 0 und bewegt sich solange weiter, wie die Steigung negativ ist. Sobald sie den ersten positiven Wert erreicht, ist der erste Meßpunkt des Reaktionsfensters auch der 1. Wert, der bei der Berechnung von V_{MaxLin} berücksichtigt werden kann.

### b) Suche von V_{MaxLin}

Mit dem im Vorlauf ermittelten Hauptlauf-Reaktionsfenster (t_{Lin}) wird über den gesamten Verlauf nach der maximalen Reaktionsgeschwindigkeit gesucht. Ist (t_{Lin}) größer als die tatsächliche Reaktionsdauer, wird die Geschwindigkeit über die gesamte Reaktion (vom Minimum bis zum Ende der Reaktionszeit) gemittelt.

Mit der so ermittelten maximalen Reaktionsgeschwindigkeit V_{MaxLin} werden die Referenzkurven erstellt, bzw. mit Hilfe der Referenzkurve Konzentrationen berechnet.

### Beispiel 2

### V_{MaxLin}-Verfahren mit Integralauswertung beim Ferritin-Test

Das Beispiel benutzt dieselben Meßwertpaare von Ferritin wie das Beispiel 1.

### a) Minimumerkennung

Die Minimumerkennung wird so durchgeführt, wie es im Beispiel 1 unter 2 a) beschrieben ist. Es wird bei der niedrig konzentrierten Probe (Tab. 1) also wieder ein Minimum bei 38 Sekunden und bei der hoch konzentrierten Probe bei 0 Sekunden gefunden. Alle Meßpunkte vor dem Minimum werden nun nicht mehr berücksichtigt.

### b) Polynom-Anpassung

An die Kurve hinter dem Minimum wird ein Polynom 3. Grades angepaßt und davon die 1. Ableitung gebildet (siehe auch Fig. 5). Das Maximum der 1. Ableitung liegt bei der niedrig konzentrierten Probe bei 153 Sekunden und bei der hoch konzentrierten Proben bei 87 Sekunden.

### c) Finden des Auswertebereiches

Unterhalb des Maximums der 1. Ableitung wird eine Integralfläche iterativ vergrößert, bis der Grenzwert, in diesem Beispiel 100, erreicht ist (siehe auch Firg. 5). Dabei wird die Fläche links und rechts vom Zeitpunkt des Maximums der 1. Ableitung getrennt vergrößert., bis die Sollfläche erreicht ist. Auf diese Weise ergibt sich das Reaktionszeitfenster. Ist die Sollfläche auf der rechten Seite bis zum letzten Meßpunkt nicht erreicht, geht bis zum letzen Meßpunkt der rechte Auswertebereich. Ist die Sollfläche auf der linken Seite bis zum Minimum nicht erreicht, geht dort das Reaktionszeitfenster bis zum Minimum.

Für die niedrige Konzentration ergibt sich dasselbe Reaktionszeitfenster wie bei dem V_{MaxLin} Verfahren mit 2 Stufen Auswertung von 38 - 360 Sekunden, für die hohe Konzentration geht das Reaktionszeitfenster von 0 bis 196 Sekunden.

### d) Ermittlung des Rohwertes

Durch lineare Regression innerhalb des Reaktionszeitfensters erhält man den Rohwert. Dieser ist bei der niedrigen Konzentration 0,123 Bit/Sekunde und bei der hohen Konzentration 23,46 Bit/Sekunde, gegenüber 23,64 Bit/Sekunde im Beispiel 1.

Dieser Wert geht entweder in eine Referenzkurve ein oder er wird zur Ermittlung einer Konzentration mit einer bestehenden Referenzkurve benutzt.

Der elementare Vorteil des V_{MaxLin}-Verfahrens gegenüber dem Stand der Technik besteht in der idealen Anpassung des Reaktionsfensters an die jeweilige Reaktionskinetik durch das zweistufige Verfahren oder das Integralverfahren, das empirisch festgelegte, testspezifische Parameter verwendet. Bei Bedarf könnten diese sogar Chargen-spezifisch festgelegt werden.

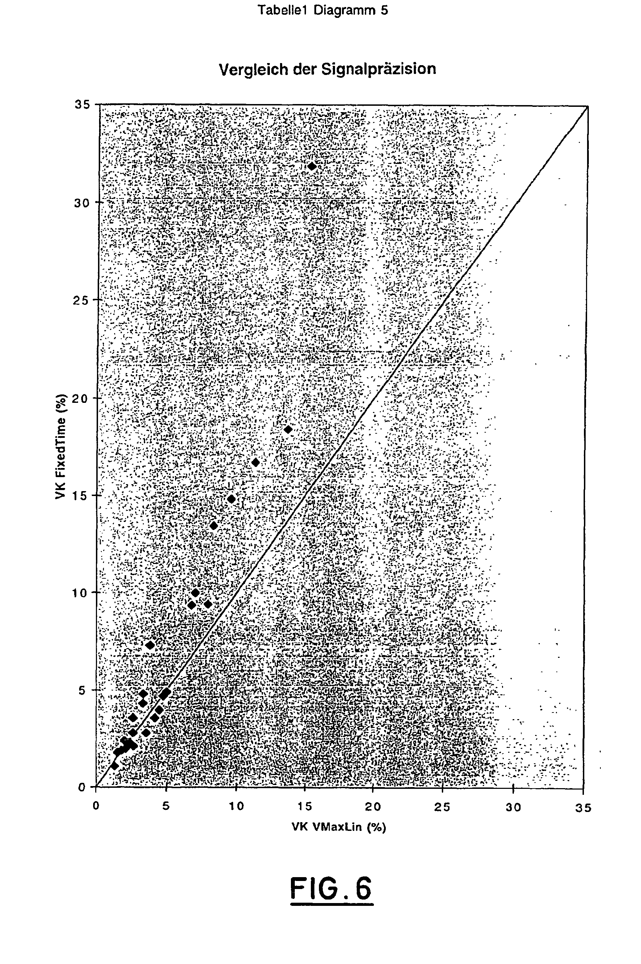

Durch die sehr spezifische Anpassung ist gewährleistet, daß jeweils eine maximale Zahl von Meßpunkten zur Auswertung herangezogen werden. Dieser Vorteil zeigt sich besonders, wenn das Signal/Rausch-Verhältnis groß ist, wie es bei den Latex-verstärkten Testen am BNII und dort gerade bei niedrigen Analyt-Konzentrationen der Fall ist. Im Vergleich mit der Fixed Time Methode, die nur die Differenz zwischen Start- und Endwerten verwendet, zeigt sich dann eine bessere Signal-Präzision (Fig. 6)

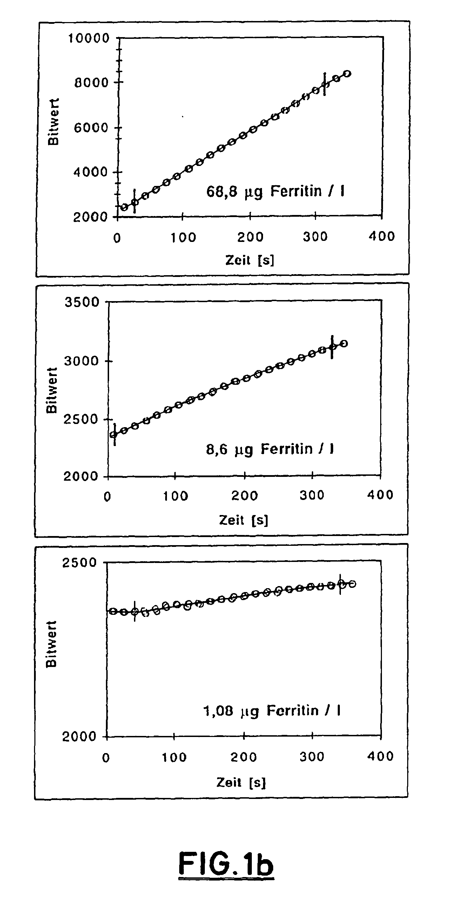

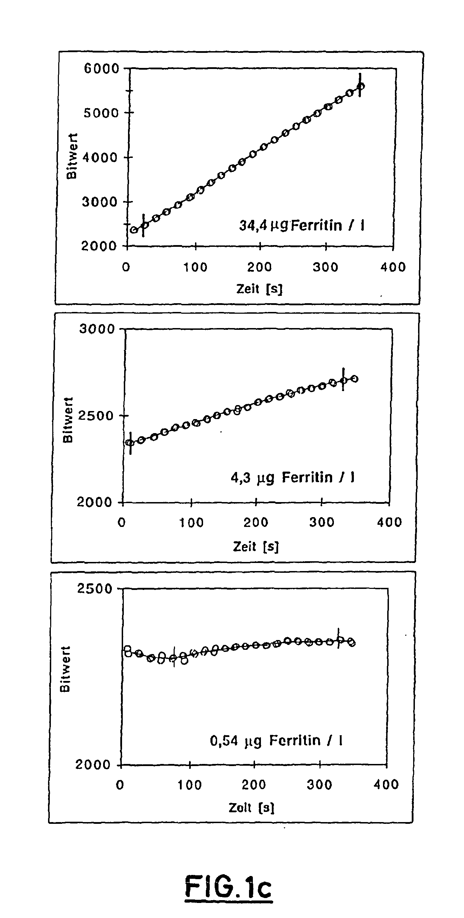

Die Peak Rate Methode ist abgestimmt auf Kinetiken, die in ihrem Verlauf eine Trennung zwischen LAG-Phase, maximaler Reaktionsgeschwindigkeit und Sättigungsbereich aufweisen (US Patent 4, 157, 871). Bei den Reaktionsverläufen der Latex-Teste am BNII (z.B. N Latex Ferritin) sind LAG-Phase und Sättigungphase oft kaum erkennbar (Fig. 1 a-1 c). Zu Beginn der Reaktion wird der erste Meßwert zu spät erfaßt und die Frequenz der Meßwertaufnahme ist auch zu gering, um die LAG-Phase noch aufzunehmen. Der Sättigungsbereich ist nach 6 Minuten noch lange nicht erreicht.

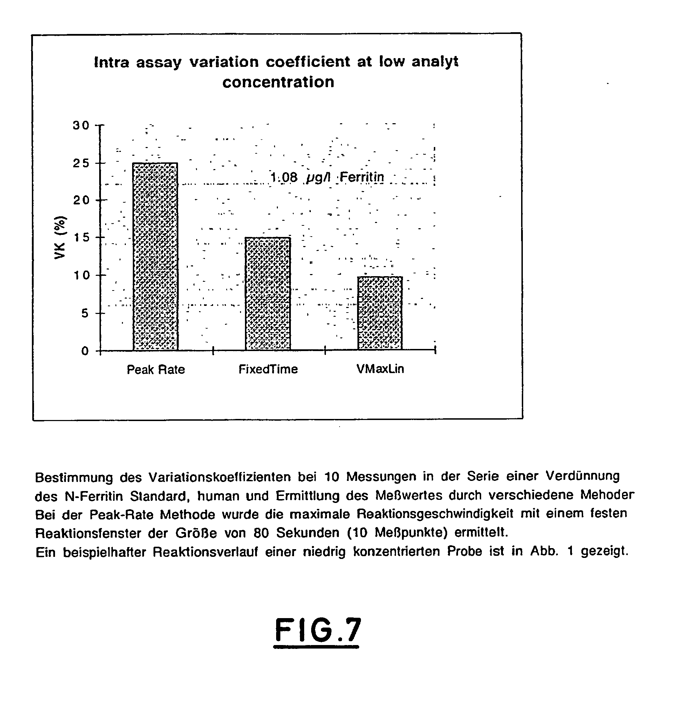

Wenn die drei Phasen nicht klar getrennt sind und zusätzlich die Frequenz der Meßwertaufnahme relativ gering ist, besteht die Gefahr, daß Peak Rate Methoden falsch zu hohe Maximalgeschwindigkeiten erfassen. Besonders bei niedrig konzentrierten Proben wie z.B. in den Fig. 1 a-1 c ist die Bestimmung einer Peak-Rate offensichtlich nicht sinnvoll, da die Reaktionsgeschwindigkeit auf niedrigem Niveau konstant ist. Das zeigt auch der Vergleich der Präzisionen mit einem kleinem Reaktionsfenster von 80 Sekunden, mit dem noch Peak-Rates erfaßt werden, und einem Reaktionsfenster von 360 Sekunden (Fig. 7).

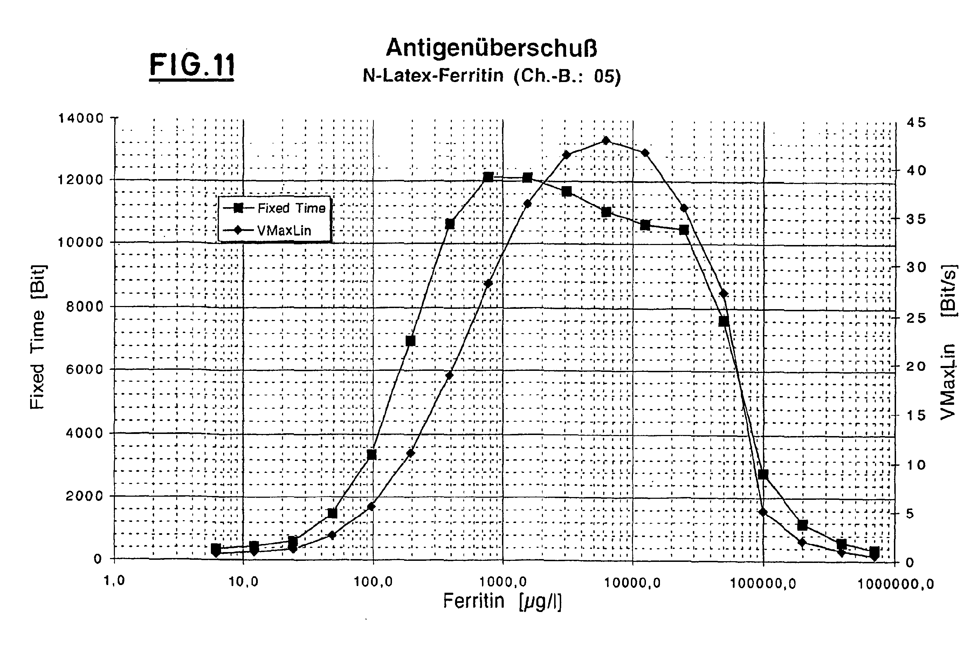

Um das neue Auswerteverfahren vergleichend zu bewerten, wurde eine Reihe von Kriterien herangezogen (Tab. 3). Bei allen Untersuchungen wurden dieselben Kinetiken jeweils nach dem herkömmlichen Fixed Time Verfahren und nach dem neuen Verfahren ausgewertet. Dabei war bei V_{MaxLin} die Testzeit auf 6 Minuten gegenüber vormals 12 Minuten verkürzt und die Referenzkurve erweitert worden. Anstelle von 5-350 µg/l ergab sich so ein Meßbereich von 2,5-700 µg/l (Probenvorverdünnung 1:5, Fig. 11)

### Tabelle 3

### Ferritin - Testeigenschaften im Vergleich der Auswerteverfahren Fixed Time und V_{MaxLin}

Bei beiden Verfahren wurde bei der Referenzkurve die LogitLog-Funktion benutzt. Jedes Ergebnis stellt den Mittelwert von 10 Messungen dar. Weitere Erklärungen sind dem Text zu entnehmen.

**Tabelle 3**

| | **Fixed Time** | **V**_{**MaxLin**} |
|---|---|---|
| **Testzeit** | 12 Minuten | 6 Minuten |
| Untere Meßbereichsgrenze | 5 µg/l | 2,5 µg/l |
| Obere Meßbereichsgrenze | 350 µg/l | 700 µg/l |
| Antigenüberschußsicherheit bis | 25000 µg/l | 50000 µg/l |
| Verdünnungsechtheit Proben | | |
| Wiederfindung 1:20 zu 1:5 | | |
| 300-340 µg/l | +16,0 % | + 6,6 % |
| 553-665 µg/l | - * | + 6,7 % |
| **Intra Assay Präzision** | | |
| Standard | | |
| 1:2,5 | 1,8 % | 1,8 % |
| 1:5 | 2,8 % | 3,6 % |
| 1:10 | 4,9 % | 5,0 % |
| 1:20 | 2,8 % | 2,7 % |
| 1:40 | 2,4 % | 2,1 % |
| 1:80 | 4,0 % | 4,5 % |
| 1:160 | 4,8 % | 3,4 % |
| 1:320 | 14,8 % | 9,6 % |
| 1:640 | 31,9 % | 15,3 % |
| Proben | | |
| Mittelwert (10) | 4,7 % | 3,4 % |
| Kontrollen | | |
| High (153 µg/l) | 2,9 % | 3,2 % |
| Low (20,2 µg/l) | 6,1 % | 4,6 % |
| **Inter Assay Präzision** | | |
| Proben | | |
| Mittelwert (5) | 2,0% | 1,8% |
| Kontrollen | | |
| High (153 µg/l) | 4,0 % | 2,6 % |
| Low (20,2 µg/l) | 4,6 % | 2,9 % |
| **Kontrollwiederfindung** | | |
| High (153 µg/l) | +2,2 % | +3,6 % |
| Low (20,2 µg/l) | +2,4 % | +3,8 % |
| **Korrelation (50 Proben):** | V_{MaxLin} = 0,951 * Fixed Time -0,2785 | |
| | R² = 0,999 | |

| | | |
|---|---|---|
| * außerhalb des Meßbereichs | | |

Die Präzision ist sowohl in der Serie (Intra Assay) als auch zwischen den Tagen (Inter Assay) bei hohen und mittleren Analytkonzentrationen gleich gut. Bei niedrigen Analyt-Konzentrationen ist die Präzision bei V_{MaxLin} deutlich besser als beim herkömmlichen Verfahren (Tab. 3).

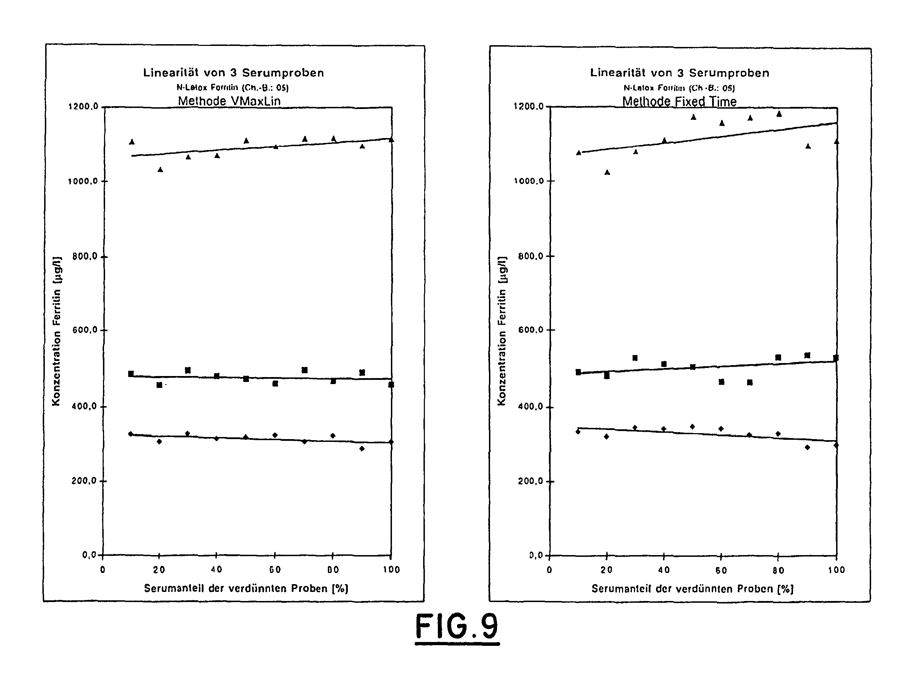

Die Verdünnungsechtheit ist deutlich besser bei V_{MaxLin} (Tab. 3, Fig. 9). Besonders bei Proben, deren Konzentration am oberen Ende des Meßbereichs liegt, führt eine Nachmessung mit der nächsthöheren Probenverdünnung (1:20) zu Werten, die um durchschnittlich 16 % höher liegen. Der gleich Versuch bei doppelt so hoch liegenden Konzentration des erweiterten Meßbereich führt mit V_{MaxLin} nur zu 7 % höheren Konzentrationen. Auch Fig. 9 zeigt die verbesserte Verdünnungsechtheit.

Die Kontrollwiederfindung ist bei beiden Auswerteverfahren ähnlich gut (Tab. 3). Dabei ist zu bedenken, daß die Sollwertermittlung mit dem Fixed time-Verfahren erfolgte, so daß eine Umstellung hier eine noch besseren Wiederfindung erwarten läßt.

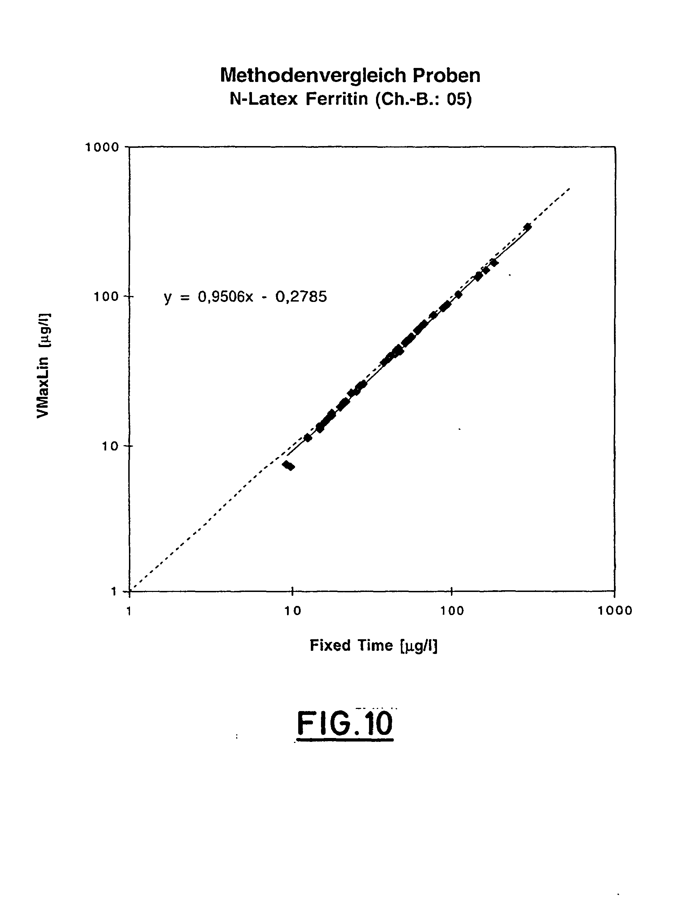

Die Korrelation der Ergebnisse beider Verfahren ist sehr gut (Fig. 10).

Die Antigenüberschußsicherheit ist bei Fixed Time time bis ca. 25000 g/l und bei V_{MaxLin} bis ca. 50000 g/l gegeben (Fig. 11).

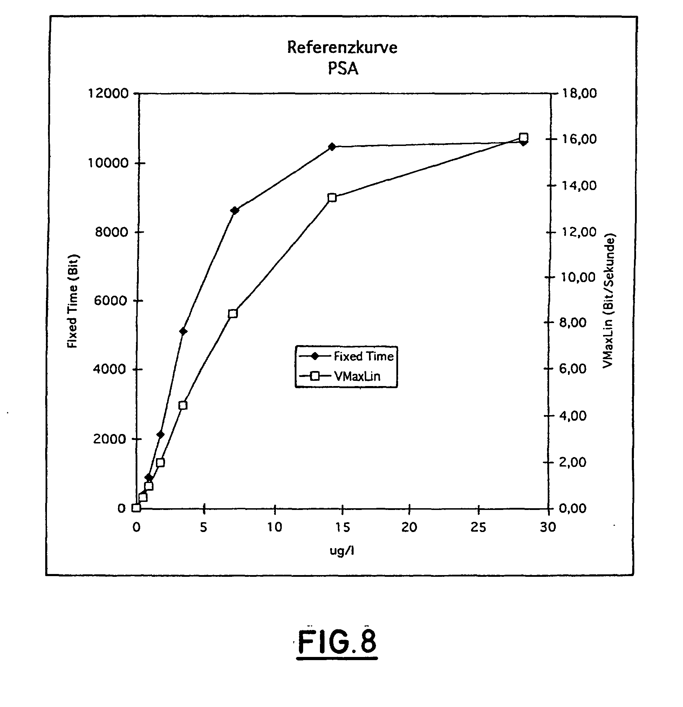

Als ein weiteres Beispiel kann der Test auf PSA (Prostata spezifisches Antigen) dienen. Wie aus der Fig. 8 ersichtlich, lassen sich über das V_{MaxLin} -Verfahren gleich gute Referenzkurven erstellen, die eine Erweiterung des Meßbereichs nach oben versprechen. Dabei beträgt die Meßzeit bei V_{MaxLin} nur 6 Minuten, gegenüber 18 Minuten bei der Fixed Time Methode.

In Tabelle 4 sind die Intra-Assay-Präzision (VK, %) von 10fach-Messungen des Standards mit Fixed Time, Integral- und 2 Stufen Auswertung bei verschiedenen Latex-verstärkten Testen gegenübergestellt.

Die beiden Varianten des V_{MaxLin}-Verfahrens sind in etwa gleichwertig und liefern bei niedrigen Konzentrationen deutlich bessere Präzisionen als die Fixed Time-Methode.

### Tabelle 4

Für die Integral-Methode wurde eine Sollfläche von 100 verwendet
n.d. - Wert wurde nicht bestimmt
.* - Mittelwert ohne Nulistand

### Meßzeiten:

FRT 6 Minuten (Integral und 2 Stufen) und 12 Minuten (Fixed-time)
PSA 12 Minuten (Integral und 2 Stufen) und 18 Minuten (Fixed-time)
BR 9 Minuten (Integral und 2 Stufen) und 12 Minuten (Fixed-time)
RF 6 Minuten (Integral und 2 Stufen) und 6 Minuten (Fixed-time)

| Verdünnung 1: | Integral | | | | 2 Stufen | | | | Fixed-time | | | |
|---|---|---|---|---|---|---|---|---|---|---|---|---|
| | FRT | PSA | BR | RF | FRT | PSA | BR | RF | FRT | PSA | BR | RF |
| 2,5 | 2,0 | | | 1,7 | 1,8 | | | 2,1 | | | | 2,2 |
| 5 | 3,4 | | | 4,2 | 3,6 | | | 3,0 | 2,8 | | | 2,5 |
| 10 | 4,9 | 2,5 | 2,6 | 5,5 | 5,0 | 2,2 | n.d. | 4,1 | 4,9 | | 2,4 | 3,7 |
| 20 | 3,0 | 4,5 | 2,9 | 3,1 | 2,7 | 5,4 | n.d. | 2,9 | 2,8 | 3,1 | 2,2 | 3,2 |
| 40 | 2,2 | 1,7 | 4,3 | 5,5 | 2,1 | 2,5 | n.d. | 5,0 | 2,4 | 1,1 | 4,1 | 5,3 |
| 80 | 4,8 | 2,7 | 6,3 | 6,4 | 4,5 | 2,8 | n.d. | 6,1 | 4,0 | 2,2 | 5,4 | 6,4 |
| 160 | 3,5 | 1,9 | 4,0 | 5,7 | 3,4 | 2,8 | n.d. | 4,2 | 4,8 | 1,8 | 4,9 | 8,5 |
| 320 | 9,7 | 2,3 | 8,2 | | 9,6 | 3,8 | n.d. | | 14,8 | 2,1 | 5,5 | |
| 640 | 15,0 | 1,7 | 7,8 | | 15,3 | 3,9 | n.d. | | | 2,0 | 13,6 | |
| Nullstand. | | 16,9 | | | | 17,9 | | | | 32,1 | | |
| MW | 5,4 | 2,5 | 5,2 | 4,6 | 5,3 | 3,3 | n.d | 3,9 | 5,2 | 2,1 | 5,4 | 4,5 |

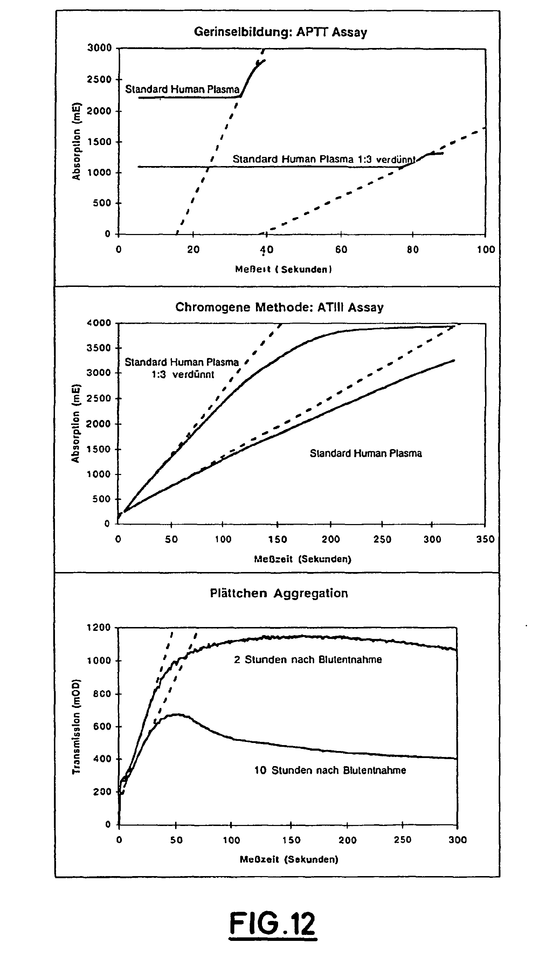

### Beispiel 3 (Fig. 12)

### VMaxLin-Verfahren mit Integralauswertung bei einem aPTT-Test

Die Auswertung erfolgt beim Behring-Originaltest so, daß der Zeitpunkt ermittelt wird, an dem eine bestimmte Schwelle der Lichtabsorption überschritten wird (Fixed absorbance-Auswertung). Es konnte gezeigt werden, daß gleichermaßen auch das V_{MaxLin} -Verfahren mit Integralauswertung verwendet werden kann. Die Signaldifferenz zwischen dem Standard und dem 1:3 verdünnten Standard (125.7 mE/Sekunde gegenüber 28.2 mE/Sekunde) ist höher als bei der Fixed absorbance-Auswertung (33.9 Sekunden gegenüber 81.4 Sekunden). Vorteilhaft ist, daß bei der V_{MaxLin} -Auswertung keine Schwelle überschritten werden muß (System: BCS, Behring-Reagenz OQGS, Behring-Testnummer: 28, Sollfläche: 10, Polynomgrad: 5, Auswertebereich: 30-100 Sekunden (Standard unverdünnt), 70-150 Sekunden (1:3 Standard), Fig. 12).

Für die V_{MaxLin} -Auswertung von Testen mit langer Lag-Phase muß der Startzeitpunkt der Auswertung variabel gehalten werden. Dazu sucht z.B. ein Algorithmus analog zur Minimumsuche nach einer positiven Grenzsteigung, deren Wert definierbar sein muß und vom Testsystem abhängt Ist die Grenzsteigung erreicht, ergibt der Beginn des Suchzeitfensters den Startzeitpunkt für die V_{maxLin} -Auswertung.

### Beispiel 4 (Fig. 12)

### VMaxLin-Verfahren mit Integralauswertung bei einem ATIII-Test.

Auch der ATIII-Test, bei dem ein chromogenes Substrat von dem nicht durch ATIII inhibierten Thrombin umgesetzt wird, kann durch VMaxLin ausgewertet werden (System: BCS, Behring-Reagenz OWWR, Behring-Testnummer: 28, Auswertebereich: 0-45 Sekunden, Sollfläche: 1, Polynomgrad: 5). Bei einer Zehnfachmessung ergaben sich in etwa gleich gute Präzisionen im Vergleich mit der herkömmlichen Auswertung (Steigung von 15-45 Sekunden).

### Beispiel 5 (Fig. 12)

### VMaxLin-Verfahren mit Integralauswertung bei einem Plättchenaggregationstest

Bei der Aggregation von Blutplättchen können sich sehr unterschiedliche Reaktionskinetiken ergeben. Im Zuge der Aggregation nimmt die Lichttransmission des Plasmas zu. Im Beispiel der Fig. 12 zeigen sich die Verläufe der Zellaggregation einer Probe 2 Stunden und 10 Stunden nach Blutentnahme. Bei beiden Verläufen findet V_{MaxLin} mit Integralauswertung sehr gut den linearen Abschnitt und ermittelt eine Aggregationsgeschwindigkeit von 22,4 für die frische und 14,5 für die alte Probe (System: BCT, Ansatz: 50 µl ADP (1.25 µM) von Sigma, St. Louis, USA, gemischt mit 150 µl Plättchen-reichem Plasma, Auswertebereich: 7-80 Sekunden, Sollfläche: 5, Polynomgrad: 5).

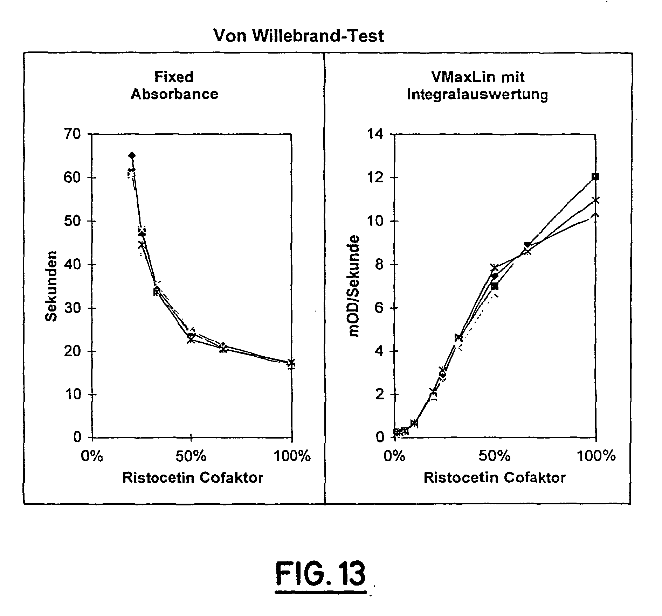

### Beispiel 6 (Fig. 13)

### VMaxLin-Verfahren mit Integralauswertung bei dem Von-Willebrand-Test

Der Von-Willebrand-Test von Behring ermittelt die von-Willebrand-Faktorabhängige Plättchenaggregation mit einem Reagenz, das fixierte Plättchen und Ristocetin enthält. Die Auswertung erfolgt beim Behring-Originaltest so, daß der Zeitpunkt ermittelt wird, an dem eine bestimmte Schwelle (100 mE) der Lichtabsorption unterschritten wird (Fixed absorbance-Auswertung). Die Referenzkurvenpunkte können bei der herkömmlichen Fixed-absorbance-Auswertung nur bis 20% Ristocetin Kofaktor-Konzentration (Standard-Humanplasma-Verdünnung) ermittel werden, weil darunter die Schwelle auf Grund der schwachen Reaktion nicht mehr unterschritten wird und damit gar kein Ergebnis mehr ermittelt werden kann. Dagegen ermöglicht V_{MaxLin} mit Integralauswertung bei den gleichen Messungen eine Referenzkurve bis ca. 5% (System: BCT, Behring-Reagenz OUBD, Behring-Testnummer 390, Auswertebereich: 7-80 Sekunden, Sollfläche: 5, Polynomgrad: 5).

## Patentansprüche

1. Verfahren zur instrumentellen Bestimmung der maximalen Reaktionsgeschwindigkeit V_{MaxLin} aus einer sich zeitbhängig mit der Reaktion ändernden Meßgröße L(t), wobei die maximale Reaktionsgeschwindigkeit V_{MaxLin} mittels eines Hauptlauf Reaktionszeitfensters in einem Reaktionsabschnitt mit der besten Linearität ermittelt wird, **dadurch gekennzeichnet, daß**
i) zunächst mittels eines vorermittelten, testspezifischen Vorlaufreaktionszeitfensters die vorläufige maximale Reaktionsgeschwindigkeit V_{MaxPre} bestimmt wird, dann
II) mittels einer vorermittelten, testspezifischen Abhängigkeit zwischen optimalem Reaktionszeitfenster und vorläufiger maximaler Reaktionsgeschwindigkeit V_{MaxPre} das Hauptlauf-Reaktionszeitfenster bestimmt wird, und dann
iii) mit Hilfe des Hauptlauf-Reaktionszeitfensters die maximale Reaktionsgeschwindigkeit V_{MaxLin} abgeleitet wird.

2. Verfahren zur instrumentellen Bestimmung der maximalen Reaktionsgeschwindigkeit V_{MaxLin} aus einer sich zeitabhängig mit der Reaktion ändernden Meßgröße L(t), wobei die maximale Reaktionsgeschwindigkeit V_{MaxLin} mittels eines Reaktionszeitfensters in einem Reaktionsabschnitt mit der besten Linearität ermittelt wird, **dadurch gekennzeichnet, daß**
i) an die Funktion L(t) ein Polynom angepaßt wird,
ii) die 1. Ableitung des gefundenen Polynoms gebildet wird,
iii) unter die 1. Ableitung eine testspezifische Fläche eingepaßt wird, wobei die Berührungspunkte der unteren Begrenzung der Fläche mit der 1. Ableitung des Polynoms den oberen und unteren Grenzwert des optimalen Reaktionszeitfensters markieren, und
iv) für das optimale Reaktionszeitfenster die maximale Reaktionsgeschwindigkeit V_{MaxLin} abgeleitet wird.

3. Verfahren gemäß einem der Ansprüche 1 und 2, wobei die maximale Reaktionsgeschwindigkeit V_{MaxLin} mittels eines Reaktionszeffensters ermittelt wird, das zusätzlich optimal für Präzision und Richtigkeit ist.

4. Verfahren gemäß einem der Ansprüche 1 bis 3, wobei die Reaktion einer Probe mit Reagenzien zu der zeitabhängigen Änderung der Meßgröße L(t) führt.

5. Verfahren gemäß Anspruch 4, in dem der Analyt in der Probe und das Reagenz Partner eines spezifischen Bindungspaares sind.

6. Verfahren gemäß Anspruch 4, in dem der Analyt in der Probe und das Reagenz Antigen und Antikörper sind.

7. Verfahren gemäß einem der Ansprüche 1 bis 6, in dem die Meßgröße die Trübung ist.

8. Verfahren gemäß einem der Ansprüche 1 bis 6, in dem die Meßgröße die Lichtstreuung ist.

9. Verfahren gemäß einem der Ansprüche 4 bis 8, in dem der Analyt in der Probe ein Plasmaprotein ist.

10. Verfahren gemäß einem der Ansprüche 4 bis 8, in dem der Analyt in der Probe ein Hapten ist.

11. Verwendung eines Verfahrens gemäß einem der Ansprüche 1 bis 10 in einem Verfahren der Gerinnungsanalytik.

## Claims

1. Method for instrument determination of the maximum reaction rate V_{MaxLin} from a measured variable L(t) which varies as a function of time with the reaction, with the maximum reaction rate V_{MaxLin} being determined by means of a main reaction time window in a reaction section with the best linearity, **characterized in that**
i) the initial maximum reaction rate, V_{MaxPre} is determined first of all by means of a previously determined, test-specific initial reaction time window, then
ii) the main reaction time window is determined by means of a previously determined, test-specific relationship between the optimum reaction time window and the initial maximum reaction rate V_{MaxPre}, and then
iii) the maximum reaction rate V_{MaxLin} is derived with the aid of the main reaction time window.

2. Method for instrument determination of the maximum reaction rate V_{MaxLin} from a measured variable L(t) which varies as a function of time with the reaction, with the maximum reaction rate V_{MaxLin} being determined by means of a reaction time window in a reaction section with the best linearity, **characterized in that**
i) a polynomial is matched to the function L(t),
ii) the first derivative is formed of the polynomial found,
iii) a test-specific area is fitted under the first derivative, the contact points of the lower boundary of the area with the first derivative of the polynomial marking the upper limit and the lower limit of the optimum reaction time window, and
iv) the maximum reaction rate V_{MaxLin} is derived for the optimum reaction time window.

3. Method according to one of Claims 1 and 2, with the maximum reaction rate V_{MaxLin} being determined by means of a reaction time window which is additionally optimal for precision and correctness.

4. Method according to one of Claims 1 to 3, with the reaction of a sample with reagents leading to the time-dependent change in the measured variable L(t).

5. Method as claimed in claim 4 in which the analyte in the sample and the reagent are partners in a specific bonding pair.

6. Method as claimed in claim 4 in which the analyte in the sample and the reagent are antigen and antibody.

7. Method as claimed in one of claims 1 to 6 in which the measured variable is the turbidity.

8. Method as claimed in one of claims 1 to 6 in which the measured variable is the light scatter.

9. Method as claimed in one of claims 4 to 8 in which the analyte in the sample is a plasma protein.

10. The method as claimed in one of claims 4 to 8 in which the analyte in the sample is a Haptene.

11. Use of the method as claimed in one of claims 1 to 10 in a method for clotting analysis.

## Revendications

1. Procédé pour la détermination instrumentale de la vitesse de réaction maximale V_{maxLin} à partir d'une valeur mesurée L(t) dépendant du temps pendant la réaction, la vitesse de réaction maximale V_{MaxLin} étant définie au moyen d'une fenêtre de temps de réaction de la phase principale dans une partie de la réaction présentant la meilleure linéarité, **caractérisée en ce que**
i) on détermine tout d'abord au moyen d'une fenêtre de temps de réaction de la phase initiale prédéfinie la vitesse de réaction maximale initiale V_{MaxPre}, puis
ii) on détermine la fenêtre de temps de réaction de la phase principale au moyen d'une dépendance prédéfinie spécifique au test entre la fenêtre de temps de réaction optimale et la vitesse de réaction maximale initiale V_{MaxPre}, puis
iii) on déduit la vitesse de réaction maximale V_{MaxLin} au moyen de la fenêtre de temps de réaction de la phase principale.

2. Procédé pour la détermination instrumentale de la vitesse de réaction maximale V_{maxLin} à partir d'une valeur mesurée L(t) dépendant du temps pendant la réaction, la vitesse de réaction maximale V_{MaxLin} étant définie au moyen d'une fenêtre de temps de réaction dans une partie de la réaction présentant la meilleure linéarité, **caractérisée en ce que**
i) un polynôme est adapté à la fonction L(t),
ii) on forme la première dérivée du polynôme trouvée
iii) une surface spécifique au test est adaptée sous la première dérivée, les points de contact entre la limite inférieure de la surface et la première dérivée du polynôme marquant la limite supérieure et la limite inférieure de la fenêtre de temps de réaction optimale, et
iv) on déduit la vitesse de réaction maximale V_{MaxLin} pour la fenêtre de temps de réaction optimale.

3. Procédé selon une des revendications 1 et 2, la vitesse de réaction maximale V_{MaxLin} étant définie au moyen d'une fenêtre de temps de réaction qui est en outre optimale pour la précision et l'exactitude.

4. Procédé selon une des revendications 1 à 3, dans lequel la réaction d'un échantillon avec des réactifs aboutit à une variation en fonction du temps de la valeur mesurée L(t).

5. Procédé selon la revendication 4, dans lequel l'analyte dans l'échantillon et le partenaire réactionnel sont une paire de liaison spécifique.

6. Procédé selon la revendication 4, dans lequel l'analyte dans l'échantillon et le réactif sont un antigène et un anticorps.

7. Procédé selon une des revendications 1 à 6, dans lequel la valeur mesurée est la turbidité.

8. Procédé selon une des revendications 1 à 6, dans lequel la valeur mesurée est la dispersion de la lumière.

9. Procédé selon une des revendications 4 à 8, dans lequel l'analyte dans l'échantillon est une protéine plasmatique.

10. Procédé selon une des revendications 4 à 8, dans lequel l'analyte dans l'échantillon est un haptène.

11. Utilisation d'un procédé selon une des revendications 1 à 10 dans un procédé d'analyse de la coagulation.
